# EUROPEAN PATENT SPECIFICATION

(11) **EP 0 878 206 B1**
(45) Date of publication and mention of the grant of the patent: **02.01.2003**
(21) Application number: 98107717.5
(22) Date of filing: 28.04.1998
(51) Int. Cl.: A61M 5/32, C09D 183/08, C09D 183/04

(54) **Syringe needle**
Spritzennadel
Aiguille de séringue

(30) Priority: 12.05.1997 JP 12126497
(43) Date of publication of application: 18.11.1998
(73) Proprietor: Nipro Corporation, Kita-ku, Osaka-shi (JP)
(72) Inventor: Arimatsu, Yoshikazu, Ootsu-shi, Shiga-ken 520-0103 (JP); Nizuka, Takeshi, Ootsu-shi, Shiga-ken 520-0813 (JP)
(74) Representative: Weisert, Annekäte, Dipl.-Ing. Dr.-Ing.

(56) References cited:
- EP-A- 0 491 547
- EP-A- 0 494 648
- EP-A- 0 627 474
- EP-A- 0 650 699

## Description

The present invention relates to a syringe needle with its surface coated with polyorganosiloxanes.

### PRIOR ART

An art of treating the metal surface with a silicone compound for reducing the surface resistance has hitherto been practised. In Japanese Patent Publication No. (Tokko-sho) 46-3627, for example, is disclosed a metal cutting blade having on its surface an adhesive coating substance which comprises a copolymer of siloxane units having amino groups and organosiloxane units. When this layer of adhesive coating is applied to a syringe needle, the coating substance is peeled off if the injection is repeatedly attempted. Such repetition occurs when a liquid medicine is introduced into a syringe tube from its needle penetrated through the rubber stopper of vial bottle and then injected into the human body through the needle. Since in this needle the piercing resistance is increased due to gamma irradiation for sterilization, hence an effective sterilizing method other than gamma ray irradiation has to be used.

Meanwhile, Japanese Patent Publication No. (Tokko-sho) 61-35870 discloses a syringe needle coated with a material prepared by a reaction between a reaction product of a silane containing an amino group and a silane containing an epoxy group and polydiorganosiloxane having silanol groups at a terminal end and then cured at room temperature or by heating at 100∼150°C. The coating has excellent curing characteristics because it contains silane having an epoxy group. However, the piercing resistance of the needle cannot be reduced sufficiently to reduce a patient's pain because the coating is too hard.

Further, Japanese Laid-Open Patent No. (Tokkai-hei) 7-178159 discloses the coating of a syringe needle with a coating agent comprising a specific amino group-containing polyorganosiloxane and a specific polyorganosiloxane, and treating the surface by a curing method including gamma-ray irradiation. Although the piercing resistance of the syringe needle is reduced when the coating is cured by gamma-ray irradiation, the resistance is not necessarily reduced by methods other than gamma-ray irradiation. Therefore, the sterilization method is limited to gamma-ray irradiation.

The present invention is for solving the aforementioned problems and is aimed to provide a syringe needle with reduced piercing resistance, which resistance is not increased even when piercing is repeated, and a piercing needle with no limitation in sterilizing methods.

After intensive research for solving such conventional defects the present inventors discovered that the aforementioned problem is solved by a syringe needle coated on its outside with a mixture of
(1) a first polyorganosiloxane (hereinafter referred to as a first polyorganosiloxane) selected from the group consisting of
   (i) a reaction product of (a) a polyorganosiloxane having amino groups at both terminal ends and alkoxysilyl groups with (b) an epoxy group-containing alkoxysilane and
   (ii) a reaction product of (a) a polyorganosiloxane having silanol groups at both terminal ends with (b) another reaction product of an organosilane having amino group at the terminal end with an epoxy group-containing alkoxysilane and
(2) a second non-reactive polyorganosiloxane, said second polyorganosiloxane having a lower average degree of polymerization of siloxane part than that of said first polyorganosiloxane.

It is preferred that the mean degree of polymerization of siloxane part of the first polyorganosiloxane claimed in claim 1 is 10 to 10,000. Also preferred is that the amount of the non-reactive polyorganosiloxane in the mixture is 1/10 to 7/10 by weight of the amount of polyorganosiloxane.

The first polyorganosiloxane (1) is synthesized from a polyorganosiloxane having silanol groups at both terminal ends. That is, reaction is carried out in two stages in a solvent as follows. In the first stage, amino group-containing alkoxysilane is reacted with polyorganosiloxane having silanol groups at both terminal ends to produce a polyorganosiloxane having amino groups at both terminal ends and alkoxysilyl groups. In the second stage, the reaction product of the first stage is reacted with epoxy group-containing alkoxysilane. In the first stage, silanol group of polyorganosiloxane reacts with alkoxysilyl group of amino group-containing alkoxysilane. In the second stage, mainly amino group of the polyorganosiloxane having amino groups at both terminal ends reacts with epoxy group of epoxy group-containing alkoxysilane to produce the first polyorganosiloxane (1).

As an amino group-containing alkoxysilane may be cited N-β-(aminoethyl) γ-aminopropylmethyldimethoxysilane, N-β-(aminoethyl) γ-aminopropyltriethoxysilane, γ-aminopropylmethoxysilane, γ-aminopropylmethyldiethoxysilane, γ-aminopropyltriethoxysilane etc.

As epoxy group-containing alkoxysilane may be cited γ-glycidoxypropyltrimethoxysilane, γ-glycidoxypropylmethyldiethoxysilane, γ-glycidoxypropyltriethoxysilane, β-(3,4-epoxy-cyclohexyl)ethyltrimethoxysilane and the like.

Instead of the reaction described above, amino group-containing alkoxysilane can be reacted with epoxy group-containing alkoxysilane in the first stage of preparation and then this reaction product may be reacted with polyorganosiloxane having silanol groups at both terminal ends in the second stage. In this case, in the first stage amino group of amino group-containing alkoxysilane and epoxy group of epoxy group-containing alkoxysilane are reacted. In the second stage, alkoxysilyl groups of the reaction product of the first stage react with terminal silanol groups of the polyorganosiloxane having silanol groups at both terminal ends to produce the first polyorganosiloxane (1). Anyway, the desirable product can be obtained by any other known methods.

The resulting product is mixed with the second non-reactive polyorganosiloxane (2) and the mixture is diluted with a solvent to about 5% by weight of nonvolatile content.

The mean degree of polymerization of the siloxane part of the second non-reactive polyorganosiloxane (2) is less than that of the siloxane part of the first polyorganosiloxane (1). If the mean degree of polymerization of the second non-reactive polyorganosiloxane (2) is higher than that of siloxane part of the polyorganosiloxane (1), it is difficult for the cross-linking of polyorganosiloxane to progress, and a good cross-linked polymer is not formed. And if the polymer is applied to the needle, the resulting syringe needle is high in piercing resistance and is inferior in piercing properties when piercing attempt is repeated.

A coated syringe needle of the present invention is prepared by repeatedly dipping a needle in a coating solution, i.e., a diluted mixture of first polyorganosiloxane (1) and non-reactive polyorganosiloxane (2), and is left to stand at room temperature.

While the needle is left at room temperature, alkoxysilyl groups attach to the surface of the needle by condensation reaction and the alkoxysilyl groups are condensed with each other to form cross-linked polymer. This kind of, reaction may be enhanced by addition of proper organic acid such as acetic acid. Also the reaction temperature may be raised to about 100°C for about 1 hour or the time of heating may be extended as necessary. When left at room temperature, the syringe needle has deposited thereon a layer of alkoxy groups to form a cross-linked film. The second non-reactive polyorganosiloxane (2) exists in the cross-linked polymer without participating in the reaction and provides good lubricating property to the needle.

That is, the syringe needle is coated with a mixture of first polyorganosiloxane (1) and second polyorganosiloxane (2), the mean degree of polymerization of siloxane parts of which is less than that of the first polyorganosiloxane. As a syringe needle there can be used an injection needle, a winged needle, a retained needle or the like, which has no particular limitation.

In a mixture of a first polyorganosiloxane (1) and a second non-reactive polyorganosiloxane (2), the mean degree of polymerization of the siloxane part of the first polyorganosiloxane (1) is preferred to be 10 to 10,000. If the polymerization degree of siloxane part is less than 10, it is difficult to cross-link with the second non-reactive polyorganosiloxane (2) and favorable cross-linking units may be not formed. Still more preferably, it is in a range of 10 to 1,000. It is preferred that the amount of the second non-reactive polyorganosiloxane (2) in said mixture is 1/10 to 7/10 by weight of the amount of polyorganosiloxane (1). If it is less than 1/10 by weight, piercing resistance tends to be increased. If the amount is more than 7/10, the syringe needle has inferior piercing properties.

The syringe needle coated with such cross-linked polymer has not only reduced piercing resistance but also a stability of the coating for repeated use of the needle. That is, the coating is not peeled off easily by repeated use of the needle. Changes of properties including piercing property and discoloration with time of the needle of the invention do not occur. Further, since it is a preferred cross-linked polymer firmly attached to the metal surface of the needle, there is no risk of the coating being eluted into blood. Since the coated polymer has been cured sufficiently, gamma ray irradiation exposed on the needle does not cause more curing, and also the needle is contacted with ethylene oxide gas, the piercing resistance is decreased rather than increased (see Table 1). Thus, the sterilizing method for the syringe needle of the present invention is not limited to irradiation.

Examples of the invention will be specified hereinafter.

### [Example 1]

5 weight parts of silicone compound having amino groups at both terminal ends and alkoxysilyl groups (MDX 4-4159, Dow Corning Inc., mean degree of polymerization: ca. 70) and 0.2 weight parts of γ-glycidoxypropyl methyldimethoxysilane (KBM-402, manufactured by Shin-etsu Chemical Co., Ltd.) were reacted in 10 weight parts of toluene for 3 hours at 80°C. After completion of the reaction, the colorless and transparent coating solution was obtained with addition of 2.5 weight parts of polydimethylsiloxane having a viscosity of 50cSt (KF-96, 50cSt, manufactured by Shin-etsu Chemical Co., Ltd., mean degree of polymerization: ca. 50) and 82.3 weight parts of dichloropentafluoropentane (AK-225, manufactured by Asahi Glass Co., Ltd.). A 21G injection needle was dipped in this coating solution and heat treatment was conducted for 1 hour at 100°C.

The syringe needle thus obtained was pricked vertically into a natural rubber sheet 1.5 mm in thickness, 30 in hardness at a crosshead speed of 100 mm/min. and the resistance value was measured by a universal tester (AG-500 of Shimadzu Corp.). The resistance when the edge of the needle has penetrated through the natural rubber sheet (first and fifth attempts) and the degree of adherence of silicone on the sheet surface are shown in Table 1.

### [Example 2]

0.1 weight part of γ-aminopropyltriethoxysilane (KBE-903, manufactured by Shin-etsu Chemical Co., Ltd.) and 0.1 weight part of γ-glycidoxypropylmethyldimethoxysilane (KBM-402, manufactured by Shin-etsu Chemical Co., Ltd.) were reacted for 3 hours at 80°C. This reaction product was added to a mixture of 3 weight parts of polydimethylsiloxane (mean degree of polymerization: ca. 300) having silanol groups at both terminal ends and 10 weight parts of toluene, and were reacted for 12 hours at 80°C. After completion of reaction, 3 weight parts of polydimethylsiloxane having a viscosity of 350cSt (DC-360, 350cSt, Dow Corning Corp., mean degree of polymerization: ca. 200), 5 weight parts of n-decane and 78.8 weight parts of dichloropentafluoropropane were added to obtain a colorless, transparent coating solution. A 21G syringe needle was dipped in the coating solution and heat-treated for 1 hour at 100°C. The syringe needle thus obtained was tested in the same way as in Example 1. Obtained results are shown in Table 1.

### [Example 3]

A 21G syringe needle was dipped in the same coating solution as in Example 2 and sterilized with ethylene oxide gas instead of 1 hour heat treatment at 100°C. The syringe needle thus treated was tested in the same way as in Example 1, and the results are shown in Table 1.

### [Example 4]

A 21G syringe needle was dipped in the same coating solution as in Example 2 and sterilized by gamma ray irradiation instead of heating for 1 hour at 100°C. The syringe needle thus prepared was tested in the same way as in Example 1, and the results are shown in Table 1.

### [Comparative Example 1]

Instead of polydimethylsiloxane having a viscosity of 50cSt used in Example 1, polydimethylsiloxane having a viscosity of 12,500cSt (DC-360, 12,500cSt, Dow Corning Inc., mean polymerization degree: ca. 850) was used. The injection needle thus obtained was tested in the same way as in Example 1 and the results are shown in Table 1.

### [Comparative Example 2]

Instead of polydimethylsiloxane having a viscosity of 350cSt, polydimethylsiloxane having a viscosity of 12,500cSt (DC-360, 12,500cSt, Dow Corning Inc., mean degree of polymerization: ca 850) was used, and the results are shown in Table 1.

| | Piercing resistance (g) | | Aherence of silicone |
|---|---|---|---|
| | 1st time | 5th time | |
| Example 1 | 11.3 | 11.5 | Minor |
| Example 2 | 9.1 | 9.7 | Very minor |
| Example 3 | 8.5 | 9.2 | Very minor |
| Example 4 | 9.3 | 9.5 | Very minor |
| Comp.example 1 | 14.7 | 23.5 | Very major |
| Comp.example 2 | 11.5 | 17.3 | Major |

As is apparent from the results shown in Table 1, the syringe needle of the comparative examples is higher in piercing resistance, and the resistance increased after repeated piercing. The amount of silicone affixed to the flat rubber was also significant with the syringe needles of the comparative examples. The syringe needle of the present invention has low piercing resistance, and the resistance does not increase by repeated piercing. The amount of silicone affixed to the flat rubber was less than that of the needles of the comparative examples. Further, the piercing resistance does not increase by sterilization with ethylene oxide gas or by gamma ray irradiation.

### [Effect of the invention]

According to the present invention, a syringe needle is provided with a decreased piercing resistance, and its resistance does not increase by repeated piercing. Further, means for sterilizing the needle is not limited.

## Claims

1. A syringe needle coated on its outside with a mixture of
(1) a first polyorganosiloxane selected from the group consisting of
(i) a reaction product of (a) a polyorganosiloxane having amino groups at both terminal ends and alkoxysilyl groups with (b) an epoxy group-containing alkoxysilane and
(ii) a reaction product of (a) a polyorganosiloxane having silanol groups at both terminal ends with (b) another reaction product of an organosilane having amino group at the terminal end with an epoxy group-containing alkoxysilane and
(2) a second non-reactive polyorganosiloxane, said second polyorganosiloxane having a lower average degree of polymerization of siloxane part than that of said first polyorganosiloxane.

2. A syringe needle according to claim 1, wherein the average degree of polymerization of siloxane part of the first polyorganosiloxane claimed in claim 1 is 10 to 10,000.

3. A syringe needle according to claim 1 or 2, wherein the amount of said non-reactive polyorganosiloxane (2) in said mixture is 1/10 to 7/10 by weight of the amount of polyorganosiloxane (1).

## Patentansprüche

1. Spritznadel, beschichtet an ihrer Außenseite mit einem Gemisch aus (1) einem ersten Polyorganosiloxan, ausgewählt aus der Gruppe bestehend aus:
(i) einem Reaktionsprodukt aus (a) einem Polyorganosiloxan mit Aminogruppen an beiden terminalen Enden und Alkoxysilylgruppen mit (b) einem Epoxygruppe-enthaltenden Alkoxysilan und (ii) einem Reaktionsprodukt aus (a) einem Polyorganosiloxan mit Silanolgruppen an beiden terminalen Enden mit (b) einem anderen Reaktionsprodukt aus einem Organosilan mit einer Aminogruppe am terminalen Ende mit einem Epoxygruppe-enthaltenden Alkoxysilan und (2) einem zweiten nichtreaktiven Polyorganosiloxan, wobei das zweite Polyorganosiloxan einen niedrigeren durchschnittlichen Polymerisationsgrad des Siloxanteils besitzt als das erste Polyorganosiloxan.

2. Spritznadel nach Anspruch 1, wobei der durchschnittliche Polymerisationsgrad des Siloxanteils des ersten Polyorganosiloxans, wie in Anspruch 1 beansprucht, 10 bis 10000 beträgt.

3. Spritznadel nach Anspruch 1 oder 2, wobei die Menge an nichtreaktivem Polyorganosiloxan (2) in dem Gemisch 1/10 bis 7/10, ausgedrückt durch das Gewicht, bezogen auf die Menge des Polyorganosiloxans (1), beträgt.

## Revendications

1. Aiguille de seringue revêtue sur son extérieur d'un mélange de (1) un premier polyorganosiloxane choisi dans le groupe composé de
(i) un produit de réaction de (a) un polyorganosiloxane ayant des groupements amino aux deux extrémités terminales et des groupements alkoxysilyl avec (b) un alkoxysilyl contenant un groupement époxy et (ii) un produit de réaction de (a) un polyorganosiloxane ayant des groupements silanol aux deux extrémités terminales avec (b) un autre produit de réaction d'un organosilane ayant un groupement amino à l'extrémité terminale avec un alkoxysilane contenant un groupement époxy et (2) un deuxième polyorganosiloxane non-réactif, ledit deuxième polyorganosiloxane ayant un degré moyen de polymérisation de la partie siloxane inférieur à celui dudit premier polyorganosiloxane.

2. Aiguille de seringue selon la revendication 1, dans laquelle le degré moyen de polymérisation de la partie siloxane du premier polyorganosiloxane selon la revendication 1 est compris entre 10 et 10 000.

3. Aiguille de seringue selon la revendication 1 ou 2, dans laquelle la quantité dudit polyorganosiloxane non-réactif (2) dans ledit mélange représente entre 1/10 et 7/10 en poids de la quantité de polyorganosiloxane (1).
